# EUROPÄISCHE PATENTSCHRIFT

(11) **EP 4 366 790 B1**
(45) Veröffentlichungstag und Bekanntmachung des Hinweises auf die Patenterteilung: **07.05.2025**
(21) Anmeldenummer: 23712223.9
(22) Anmeldetag: 16.03.2023
(51) Int. Cl.: A61L 2/26

(54) **STERILISIERBEHÄLTER UND SYSTEM**
STERILISING CONTAINER AND SYSTEM
RÉCIPIENT DE STÉRILISATION ET SYSTÈME

(30) Priorität: 17.03.2022 DE 102022106280
(43) Veröffentlichungstag der Anmeldung: 15.05.2024
(73) Patentinhaber: Aesculap AG, 78532 Tuttlingen (DE)
(72) Erfinder: JANSEN-TROY, Arne, 78333 Stockach (DE); HENKE, Matthias, 78048 Villingen-Schwenningen (DE); KIESSLING, Daniel, 78502 Villingen-Schwenningen (DE)
(74) Vertreter: Winter, Brandl - Partnerschaft mbB
(86) Internationale Anmeldenummer: PCT/EP2023/056836
(87) Internationale Veröffentlichungsnummer: WO 2023/175120

(56) Entgegenhaltungen:
- EP-A1- 2 090 324
- EP-A2- 3 194 150
- WO-A1-2022/012751
- IT-A1- 202000 009 004
- US-A1- 2021 379 227

## Beschreibung

### Technisches Gebiet

Die vorliegende Offenbarung bezieht sich auf einen Sterilisierbehälter für medizinische Zwecke gemäß dem Oberbegriff des Anspruchs 1, insbesondere auf einen Sterilisierbehälter, dessen Deckel an einem Bodenelement festlegbar ist, sodass der Deckel in seinem geschlossenen Zustand zusammen mit dem Bodenelement einen Innenbereich des Sterilisierbehälters definiert, von welchem aus zumindest eine fluidmechanische Verbindung zu einer Umgebung des Sterilisierbehälters gebildet ist, die von einem Medienaustauschmechanismus, gesteuert von einer Steuerungseinrichtung, wahlweise geöffnet und geschlossen werden kann. Die vorliegende Offenbarung bezieht sich des Weiteren auf eine Steuerungsvorrichtung zum externen Steuern der Steuerungseinrichtung des Sterilisierbehälters.

### Stand der Technik

Ein herkömmlicher Sterilisierbehälter ist beispielweise aus der WO 2020 / 074 657 A1 bzw. US 2021 / 0 379 227 A1 bekannt und weist ein Bodenelement und einen Deckel auf. Der Deckel kann an dem Bodenelement festgelegt werden, sodass ein Innenbereich des Sterilisierbehälters gebildet ist. In dem Bodenelement sind zwei Öffnungen vorgesehen, die jeweils eine fluidmechanische Verbindung von dem Innenbereich zu einer Umgebung darstellen. Der Sterilisierbehälter gemäß WO 2020 / 074 657 A1 weist mit elektromechanischen Aktoren ausgestattete Ventileinheiten auf, mittels derer die Öffnungen im Bodenelement gemäß Steuerungsbefehlen einer Steuereinheit geöffnet und geschlossen werden können.

An dem herkömmlichen Sterilisierbehälter ist von Nachteil, dass Instrumente nach Abschluss einer Sterilisation in dem gattungsgemäßen Sterilisierbehälter noch sehr feucht und/oder sehr heiß sein können, so dass in einem Instrumentenkreislauf unter Umständen lange Nachtrocknungsphasen einbezogen werden müssen.

Ein gattungsgemäßer Sterilisierbehälter ist aus der EP 2 090 324 A1 bekannt. Der gattungsgemäße Behälter weist einen Medienaustauschmechanismus in Form eines Ventils auf. Das Ventil ist an einem Stutzen vorgesehen und ermöglicht einen Medienaustausch zwischen dem Behälter und seiner Umgebung. Der gattungsgemäße Sterilisierbehälter weist des Weiteren eine Ventilationseinrichtung und eine Steuerungseinrichtung auf.

WO 2022 /012 751 A1 offenbart eine Desinfektionsvorrichtung. EP 3 194 150 A2 bzw. WO 2016 / 162 114 A2 offenbart im Zusammenhang mit einem Bioreaktor einen Behälter zur Aufnahme von mindestens einem biologisch aktiven Fluid. IT 2020 000 09 004 A1 offenbart einen Sterilisierberhälter mit einem Ventilator und einem demontierbaren Filter.

### Zusammenfassung der Offenbarung

Vor diesem Hintergrund ist es Aufgabe der vorliegenden Offenbarung, einen Sterilisierbehälter bereit zu stellen, der keine oder nur vergleichsweise kurze Nachtrocknungsphasen erforderlich macht.

Diese Aufgabe wird durch einen Sterilisierbehälter gemäß Anspruch 1 gelöst. Vorteilhafte Weiterbildungen sind Gegenstand der Unteransprüche.

Der offenbarungsgemäße Sterilisierbehälter ist für medizinische Zwecke ausgebildet und eignet sich insbesondere für die Aufnahme von ärztlichen oder chirurgischen Instrumenten. Der Sterilisierbehälter weist ein Bodenelement, einen Deckel, einen Medienaustauschmechanismus und eine Steuerungseinrichtung auf.

Das Bodenelement ist insbesondere wannenförmig ausgebildet.

Der Deckel ist ausgebildet, an dem Bodenelement derart angebracht werden zu können, dass der Deckel und das Bodenelement einen Innenbereich des Sterilisierbehälters definieren (geschlossener Zustand des Deckels).

Der Medienaustauschmechanismus ist ausgebildet, während des geschlossenen Zustands des Deckels zumindest eine fluidmechanische Verbindung zwischen dem Innenbereich und einer Umgebung wahlweise öffnen und schließen zu können.

Der Medienaustauschmechanismus kann ausschließlich am Deckel, ausschließlich an dem Bodenelement oder teilweise an dem Deckel und teilweise an dem Bodenelement ausgebildet sein. Die fluidmechanische Verbindung ist insbesondere eine Öffnung. Um diese Öffnung wahlweise öffnen und schließen zu können, ist insbesondere ein Ventilkörper vorgesehen, der vorzugsweise mittels eines elektromechanischen Aktors bewegt werden kann. Die Steuerungseinrichtung ist insbesondere ausgebildet, den Aktor derart steuern zu können, dass dieser den Ventilkörper in eine geschlossene Stellung bringen kann, in welcher der Ventilkörper die zumindest eine Öffnung insbesondere gas- und/oder flüssigkeitsdicht und/oder keimundurchlässig verschließt, und in eine geöffnete Stellung bringen kann, in welcher der Ventilkörper ein Strömen eines Mediums aus dem Innenbereich in eine Umgebung und/oder ein Strömen eines Mediums aus der Umgebung in den Innenbereich ermöglicht. Der Ventilkörper ist als ein keimundurchlässiger aber luft- bzw. gasdurchlässiger Filter ausgebildet. Vorzugsweise ist der Ventilkörper bzw. der Filter ausgebildet, ausgetauscht bzw. werkzeuglos ausgetauscht werden zu können.

Offenbarungsgemäß weist der Sterilisierbehälter eine Ventilationseinrichtung mit einem Ventilator auf, die ausgebildet ist, ein Medium in dem Innenbereich über den Medienaustauschmechanismus in die Umgebung des Sterilisierbehälters und/oder ein Medium in der Umgebung über den Medienaustauschmechanismus in den Innenbereich des Sterilisierbehälter fördern zu können. Anders ausgedrückt ist die den Ventilator aufweisende Ventilationseinrichtung ausgebildet, ein Medium in dem Innenbereich und/oder ein Medium in der Umgebung des Sterilisierbehälter zu dem Medienaustauschmechanismus fördern zu können. Das Medium in dem Innenbereich und/oder in der Umgebung ist insbesondere keimfreie Luft. Die Luft bzw. das Medium in dem Innenbereich ist insbesondere feucht. Die Luft bzw. das Medium in der Umgebung ist insbesondere trocken.

Die Ventilationseinrichtung weist insbesondere einen Ventilator auf, der an der Öffnung angeordnet ist. Der Ventilator kann vorzugsweise als Axialventilator oder als Radialventilator ausgebildet sein.

Die Steuerungseinrichtung des Sterilisierbehälters ist insbesondere ausgebildet, den Medienaustauschmechanismus und die Ventilationseinrichtung derart steuern zu können, dass die zumindest eine fluidmechanische Verbindung bei einer Förderung von einem Medium offen ist.

Die Steuerungseinrichtung ist offenbarungsgemäß ausgebildet, den Medienaustauschmechanismus und die Ventilationseinrichtung derart steuern zu können, dass die die fluidmechanische Verbindung bei einer Förderung eines Mediums in dem Innenbereich in die Umgebung geöffnet wird bzw. ist und die fluidmechanische Verbindung bei einer Förderung eines Mediums in der Umgebung in den Innenbereich mittels des keimundurchlässigen aber luft- bzw. gasdurchlässigen Filters geschlossen wird bzw. ist.

Der Medienaustauschmechanismus kann insbesondere ausgebildet sein, durch die Ventilationseinrichtung geöffnet werden zu können. Anders ausgedrückt kann der Ventilkörper bzw. Filter in seine geschlossene Stellung mit einer derartigen Vorspannung vorgespannt sein, die durch einen von der Ventilationseinrichtung erzeugten Unterdruck bzw. durch ein von der Ventilationseinrichtung erzeugtes Druckgefälle aufgehoben werden kann.

Durch die Ventilationseinrichtung wird in vorteilhafter Weise ein Mittel zum aktiven Austausch eines in dem Innenbereich des Sterilisierbehälters befindlichen Mediums bereitgestellt. Durch einen aktiven Austausch kann eine Nachtrocknungsphase in einem Instrumentenkreislauf verkürzt oder vermieden werden.

Gemäß einem Aspekt der Offenbarung kann der Sterilisierbehälter eine Sensoreinrichtung aufweisen. Die Sensoreinrichtung kann ausgebildet sein, einen Druck, eine Temperatur und/oder eine Feuchtigkeit in dem Innenbereich des Sterilisierbehälters und/oder in der Umgebung des Sterilisierbehälters messen zu können. Die Steuerungseinrichtung kann ausgebildet sein, den Medienaustauschmechanismus und die Ventilationseinrichtung unter Berücksichtigung eines Messwerts oder mehrerer Messwerte der Sensoreinrichtung steuern zu können. Insbesondere kann die Steuerungseinrichtung ausgebildet sein, Messwerte der Sensoreinrichtung aufzeichnen und mit in der Steuerungseinrichtung gespeicherte Vergleichswerte oder Vergleichsprofile vergleichen zu können und den Medienaustauschmechanismus und die Ventilationseinrichtung zu Zeitpunkten, die anhand der Vergleichswerte oder der Vergleichsprofile vorbestimmt sind bzw. werden, betätigen zu können.

Wird der Sterilisierbehälter mit einer Sensoreinrichtung ausgestattet, kann in vorteilhafter Weise eine automatische Steuerung eines Medienaustauschs realisiert werden.

Gemäß einem Aspekt der Offenbarung können der Medienaustauschmechanismus und die Ventilationseinrichtung ausschließlich an dem Deckel ausgebildet sein. Insbesondere können auch die Steuerungseinrichtung und gegebenenfalls auch die Sensoreinrichtung ausschließlich an dem Deckel ausgebildet sein.

Werden elektronische bzw. elektromechanische Komponenten ausschließlich an dem Deckel ausgebildet, kann im Falle eines Defekts dieser Komponenten das Bodenelement weiterverwendet werden.

Gemäß einem Aspekt der Offenbarung kann der Medienaustauschmechanismus zwei jeweils wahlweise öffen- und schließbare, fluidmechanische Verbindungen bzw. Öffnungen aufweisen und kann die Steuerungseinrichtung ausgebildet sein, die Ventilationseinrichtung derart steuern zu können, dass die Ventilationseinrichtung ein Medium in der Umgebung des Sterilisierbehälters zu einen ersten der zwei fluidmechanischen Verbindungen fördert und ein Medium in dem Innenbereich des Sterilisierbehälters zu einer zweiten der zwei fluidmechanischen Verbindung fördert. Insbesondere ist je fluidmechanischer Verbindung ein Ventilkörper bzw. ein Filter vorgesehen. Die fluidmechanischen Verbindungen sind insbesondere an zwei gegenüberliegenden Seiten des Sterilisierbehälters angeordnet.

Durch Bereitstellen von zwei fluidmechanischen Verbindungen kann in vorteilhafter Weise ein optimierter Medienaustausch realisiert werden.

Gemäß einem Aspekt der Offenbarung kann die Steuerungseinrichtung ausgebildet sein, die Ventilationseinrichtung derart steuern zu können, dass die Ventilationseinrichtung wechselweise einerseits ein Medium in der Umgebung des Sterilisierbehälters zu der ersten fluidmechanischen Verbindung fördert sowie ein Medium in dem Innenbereich des Sterilisierbehälters zu der zweiten fluidmechanischen Verbindung fördert und andererseits ein Medium in der Umgebung des Sterilisierbehälters zu der zweiten fluidmechanischen Verbindung fördert sowie ein Medium in dem Innenbereich des Sterilisierbehälters zu der ersten fluidmechanischen Verbindung fördert.

Wird der Sterilisierbehälter derart ausgebildet, dass eine Strömungsrichtung variiert werden kann, können eventuelle Totgebiete minimiert werden.

Gemäß einem Aspekt der Offenbarung kann der Sterilisierbehälter einen kabellos aufladbaren Energiespeicher aufweisen, der ausgebildet ist, die Steuerungseinrichtung, den Medienaustauschmechanismus und/oder die Ventilationseinrichtung mit Energie versorgen zu können. Der Energiespeicher kann insbesondere als Batterie bzw. galvanische Zelle oder Akkumulator ausgebildet sein. Der Energiespeicher ist insbesondere mit einer Induktionsspule ausgestattet, mittels derer ein kabelloses Laden des Energiespeichers möglich ist.

Wird der Energiespeicher derart ausgebildet, dass er kabellos aufgeladen werden kann, ist es in vorteilhafter Weise möglich, einen Energiespeicher mit geringem Energiespeichervermögen einzusetzen, ohne Abstriche bei der Funktionalität hinnehmen zu müssen.

Gemäß einem Aspekt der Offenbarung kann die Steuerungseinrichtung einen Empfänger aufweisen, der ausgebildet ist, Betätigungssignale empfangen zu können, welche die Steuerungseinrichtung veranlassen, Medium mittels der Ventilationseinrichtung aus dem Innenbereich und/oder aus der Umgebung des Sterilisierbehälters zu fördern. Der Empfänger ist insbesondere ausgebildet, Betätigungssignale kabellos empfangen zu können.

Gemäß einem Aspekt der Offenbarung kann die Steuerungseinrichtung einen Sender aufweisen, der ausgebildet ist, Statussignale senden zu können, welche eine Information über einen Status des Medienaustauschmechanismus und/oder der Ventilationseinrichtung enthalten. Der Sender ist insbesondere ausgebildet, Statussignale kabellos senden zu können.

Vorzugsweise sind der Sender und der Empfänger der Steuerungseinrichtung integral in Form eines Transceivers ausgebildet.

Durch Vorsehen eines Empfängers und/oder eines Senders kann ein Automatisierungsgrad eines Sterilisiervorganges erhöht und/oder die Überwachung des Sterilisiervorganges verbessert werden.

Eine offenbarungsgemäße Steuerungsvorrichtung ist geeignet, eine Steuerungseinrichtung eines offenbarungsgemäßen Sterilisierbehälters extern zu steuern. "Extern" heißt insbesondere, dass die Steuerungsvorrichtung nicht Bestandteil des Sterilisierbehälters ist oder nicht fest mit dem Sterilisierbehälter verbunden ist.

Die offenbarungsgemäße Steuerungsvorrichtung weist einen Sender (Sender der Steuerungsvorrichtung im Gegensatz zum Sender der Steuerungseinrichtung) auf, der ausgebildet ist, Betätigungssignale an die Steuerungseinrichtung senden zu können, und/oder weist einen Empfänger (Empfänger der Steuerungsvorrichtung im Gegensatz zum Empfänger der Steuerungseinrichtung) auf, der ausgebildet ist, Statussignale von der Steuerungseinrichtung empfangen zu können. Vorzugsweise sind der Sender und der Empfänger der Steuerungsvorrichtung integral in Form eines Transceivers ausgebildet. Insbesondere ist die Steuerungsvorrichtung ausgebildet, kabellos mit der Steuerungseinrichtung des Sterilisierbehälter kommunizieren zu können. Anders ausgedrückt, ist die Steuerungsvorrichtung insbesondere als eine Fernsteuerung für den Sterilisierbehälter ausgebildet. Insbesondere ist die externe Steuerungsvorrichtung an oder in einem Autoklav ausgebildet.

Wird eine externe Steuerungsvorrichtung bereitgestellt, kann der Automatisierungsgrad eines Sterilisiervorganges erhöht werden.

Gemäß einem Aspekt der Offenbarung kann die Steuerungsvorrichtung eine Ladeeinrichtung aufweisen, die ausgebildet ist, einen Energiespeicher eines offenbarungsgemäßen Sterilisierbehälters kabellos laden zu können. Insbesondere kann die Ladeeinrichtung eine Induktionsspule aufweisen, die mit der Induktionsspule der Steuerungseinrichtung zusammenwirken kann.

Durch Vorsehen einer Ladeeinrichtung in der Steuerungsvorrichtung kann der Sterilisierbehälter ohne separate, abseits eines Instrumentenkreislaufes ablaufende Aufladungszeiten genutzt werden.

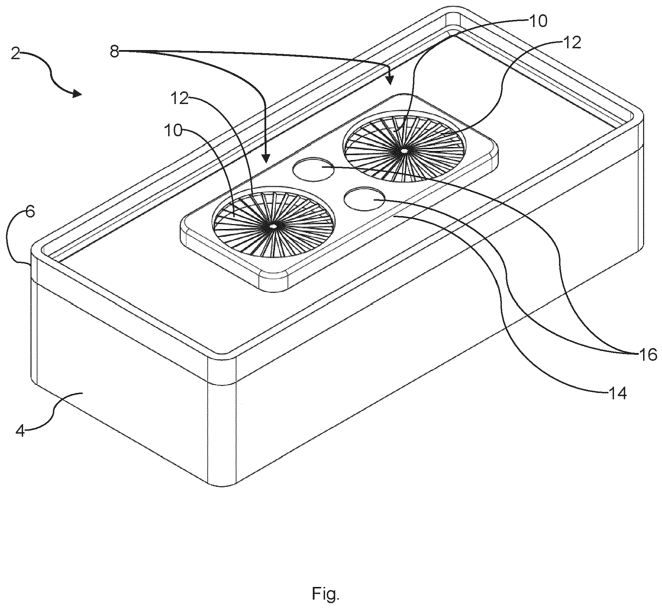

### Kurzbeschreibung der Zeichnung

Die vorliegende Erfindung wird im Folgenden anhand eines bevorzugten Ausführungsbeispiels unter Bezugnahme auf die beigefügte Figur näher beschrieben. Die Figur zeigt eine perspektivische Ansicht eines offenbarungsgemäßen Sterilisierbehälters.

### Detaillierte Beschreibung der bevorzugten Ausführungsform

Der in der Figur gezeigte offenbarungsgemäße Sterilisierbehälters 2 weist ein wannenförmiges Bodenelement 4 und einen Deckel 6 auf. Der Sterilisierbehälter 2 hat einen im Wesentlichen rechteckigen Umriss.

Die Figur zeigt den Sterilisierbehälter 2 in einem geschlossenen Zustand des Deckels 6. In diesem Zustand definieren das Bodenelement 4 und der Deckel 6 einen (nicht gezeigten) Innenbereich. Der Deckel 6 ist in dem geschlossenen Zustand über eine (nicht gezeigte) Dichtung am Umfang des Deckels 6 bzw. an freien Rändern von Seitenwänden des Bodenelements 4 mit dem Bodenelement 4 keimundurchlässig verbunden.

Im Deckel 6 sind zwei Öffnungen 8 vorgesehen, welche mittels eines Medienaustauschmechanismus öffen- und schließbar ist. Der Medienaustauschmechanismus weist zwei austauschbare, keimundurchlässige aber luftdurchlässige Filter 10 auf, welche in den Öffnungen 8 angeordnet sind. In dem in der Figur gezeigten Zustand befinden sich die Filter 10 in einer geschlossenen Stellung. In der geschlossenen Stellung der Filter 10 und in dem geschlossenen Zustand des Deckels 6 ist der Innenbereich gegenüber einer Umgebung des Sterilisierbehälters 2 keimundurchlässig verschlossen.

Oberhalb eines jeden der beiden Filter 10 bzw. an einer der Umgebung zugewandten Außenseite eines jeden der beiden Filter 10 ist jeweils ein Axialventilator 12 vorgesehen.

Jeder der beiden Axialventilatoren 12 ist von einer Steuerungseinrichtung 14 bzw. von einem Gehäuse der Steuerungseinrichtung 14 eingefasst. In der Steuerungseinrichtung 14 ist ein (nicht gezeigter) Energiespeicher vorgesehen, der ebenfalls in der Steuerungseinrichtung vorgesehene (nicht gezeigte) Elektromotoren zum Antrieb der Axialventilatoren 12 mit Energie versorgt.

An der Außenseite der Steuerungseinrichtung 14 ist zwischen den Axialventilatoren 12 eine Sensoreinrichtung 16 mit zwei Sensoren zum Messen einer Temperatur, eines Drucks und/oder einer Feuchtigkeit der Umgebung und des Innenbereichs angeordnet.

Die Steuerungseinrichtung 14 ist ausgebildet, bei Messung eines vorbestimmten Messwerts bzw. mehrerer vorbestimmter Messwerte durch die Sensoreinrichtung 16 einen der beiden Axialventilatoren 12 derart zu steuern, dass ein Druckgefälle in Richtung der Umgebung erzeugt wird, und den anderen der beiden Axialventilatoren 12 derart zu steuern, dass ein Druckgefälle in Richtung des Innenraums erzeugt wird.

Die Filter 10 sind derart ausgebildet, dass sie sich automatisch öffnen, wenn ein Druckgefälle in Richtung der Umgebung an dem jeweiligen Filter 10 anliegt, und dass sie in der geschlossenen Stellung verbleiben, wenn ein Druckgefälle in Richtung des Innenbereichs an dem jeweiligen Filter 10 anliegt. Somit wird ein Medium bzw. Luft, das bzw. die von einem Axialventilator 12 in den Innenbereich gefördert wird, von dem entsprechenden Filter 10 gefiltert. Dem gegenüber wird ein Medium bzw. Luft, das bzw. die von einem Axialventilator 12 in die Umgebung gefördert wird, nicht gefiltert.

Die Steuerungseinrichtung 14 ist ausgebildet, denjenigen der beiden Axialventilatoren 12, der Medium in den Innenbereich fördert, derart zu steuern, dass der von dem entsprechenden Filter 10 erzeugte Strömungswiderstand überwunden wird. Die Axialventilatoren 12 werden insbesondere derart gesteuert, dass ein Medium- bzw. Luftstrom in den Innenbereich gleich groß ist wie ein Medium- bzw. Luftstrom aus dem Innenbereich.

### Bezugszeichenliste

- 2: Sterilisierbehälter
- 4: Bodenelement
- 6: Deckel
- 8: Öffnung
- 10: Filter
- 12: Axialventilator
- 14: Steuerungseinrichtung
- 16: Sensoreinrichtung

## Patentansprüche

1. Sterilisierbehälter (2) für medizinische Zwecke mit
einem Bodenelement (4),
einem an dem Bodenelement (4) festlegbaren Deckel (6), der in einem geschlossenen Zustand des Deckels (6) zusammen mit dem Bodenelement (4) einen Innenbereich des Sterilisierbehälters (2) definiert,
einem Medienaustauschmechanismus,
einer Steuerungseinrichtung (14) und
einer Ventilationseinrichtung, die einen Ventilator (12) aufweist und die ausgebildet ist, ein Medium in dem Innenbereich des Sterilisierbehälter (2) zu dem Medienaustauschmechanismus fördern zu können,
**dadurch gekennzeichnet, dass**
der Medienaustauschmechanismus ausgebildet ist, in dem geschlossenen Zustand des Deckels (6) zumindest eine fluidmechanische Verbindung (8) zwischen dem Innenbereich und einer Umgebung mittels eines keimundurchlässigen aber luft- oder gasdurchlässigen Filters (10) wahlweise öffnen und schließen zu können,
die Ventilationseinrichtung (12) ausgebildet ist, ein Medium in der Umgebung des Sterilisierbehälter (2) zu dem Medienaustauschmechanismus fördern zu können, und
die Steuerungseinrichtung (14) ausgebildet ist, den Medienaustauschmechanismus und die Ventilationseinrichtung (12) derart steuern zu können, dass die zumindest eine fluidmechanische Verbindung (8) bei einer Förderung eines Mediums in dem Innenbereich in die Umgebung geöffnet ist und bei einer Förderung eines Mediums in der Umgebung in den Innenbereich mittels des keimundurchlässigen aber luft- oder gasdurchlässigen Filters (10) geschlossen ist.

2. Sterilisierbehälter (2) nach Anspruch 1, **dadurch gekennzeichnet, dass** der Sterilisierbehälter (2) eine Sensoreinrichtung (16) aufweist, die ausgebildet ist, einen Druck, eine Temperatur und/oder eine Feuchtigkeit in dem Innenbereich des Sterilisierbehälters (2) und/oder in der Umgebung des Sterilisierbehälters (2) messen zu können, und
die Steuerungseinrichtung (14) ausgebildet ist, den Medienaustauschmechanismus und die Ventilationseinrichtung (12) unter Berücksichtigung eines Messwerts oder mehrerer Messwerte der Sensoreinrichtung (16) steuern zu können.

3. Sterilisierbehälter (2) nach Anspruch 1 oder 2, **dadurch gekennzeichnet, dass** der Medienaustauschmechanismus und die Ventilationseinrichtung (12) an dem Deckel (6) ausgebildet sind.

4. Sterilisierbehälter (2) nach einem der Ansprüche 1 bis 3, **dadurch gekennzeichnet, dass**
der Medienaustauschmechanismus zwei jeweils wahlweise öffen- und schließbare, fluidmechanische Verbindungen (8) aufweist und
die Steuerungseinrichtung (14) ausgebildet ist, die Ventilationseinrichtung (12) derart steuern zu können, dass die Ventilationseinrichtung (12) ein Medium in der Umgebung des Sterilisierbehälters (2) zu einen ersten der zwei fluidmechanischen Verbindungen (8) fördert und ein Medium in dem Innenbereich des Sterilisierbehälters (2) zu einer zweiten der zwei fluidmechanischen Verbindung (8) fördert.

5. Sterilisierbehälter (2) nach Anspruch 4, **dadurch gekennzeichnet, dass** die Steuerungseinrichtung (14) ausgebildet ist, die Ventilationseinrichtung (12) derart steuern zu können, dass die Ventilationseinrichtung (12) wechselweise einerseits ein Medium in der Umgebung des Sterilisierbehälters (2) zu der ersten fluidmechanischen Verbindung (8) fördert sowie ein Medium in dem Innenbereich des Sterilisierbehälters (2) zu der zweiten fluidmechanischen Verbindung (8) fördert und andererseits ein Medium in der Umgebung des Sterilisierbehälters (2) zu der zweiten fluidmechanischen Verbindung (8) fördert sowie ein Medium in dem Innenbereich des Sterilisierbehälters (2) zu der ersten fluidmechanischen Verbindung (8) fördert.

6. Sterilisierbehälter (2) nach einem der Ansprüche 1 bis 5, **gekennzeichnet durch** einen kabellos aufladbaren Energiespeicher, der ausgebildet ist, die Steuerungseinrichtung (14), den Medienaustauschmechanismus und/oder die Ventilationseinrichtung (12) mit Energie versorgen zu können.

7. Sterilisierbehälter (2) nach einem der Ansprüche 1 bis 6, **dadurch gekennzeichnet, dass** die Steuerungseinrichtung (14) einen Empfänger aufweist, der ausgebildet ist, Betätigungssignale empfangen zu können, welche die Steuerungseinrichtung veranlassen, Medium mittels der Ventilationseinrichtung (12) aus dem Innenbereich und/oder aus der Umgebung des Sterilisierbehälters (2) zu fördern.

8. Sterilisierbehälter (2) nach einem der Ansprüche 1 bis 7, **dadurch gekennzeichnet, dass** die Steuerungseinrichtung (14) einen Sender aufweist, der ausgebildet ist, Statussignale senden zu können, welche eine Information über einen Status des Medienaustauschmechanismus und/oder der Ventilationseinrichtung (12) enthalten.

9. System mit einem Sterilisierbehälter (2) nach Anspruch 7 oder 8 und einer Steuerungsvorrichtung zum externen Steuern der Steuerungseinrichtung (14) des Sterilisierbehälters (2), **gekennzeichnet, durch** einen Sender, der ausgebildet ist, Betätigungssignale an die Steuerungseinrichtung senden zu können, und/oder einen Empfänger, der ausgebildet ist, Statussignale von der Steuerungseinrichtung (14) empfangen zu können.

10. System nach Anspruch 9 **gekennzeichnet, durch** eine Ladeeinrichtung zum kabellosen Laden eines Energiespeichers eines Sterilisierbehälter (2) nach Anspruch 6.

## Claims

1. A sterilization container (2) for medical purposes, comprising
a bottom element (4),
a lid (6) fixable to the bottom element (4) and which, in a closed state of the lid (6), together with the bottom element (4), defines an inner region of the sterilization container (2),
a media exchange mechanism,
a control device (14), and
a ventilating device, which comprises a fan (12) and which is configured to be able to convey a medium in the inner region of the sterilization container (2) to the media exchange mechanism,
**characterized in that**
the media exchange mechanism is configured to be able to selectively open and close at least one fluid-mechanical connection (8) between the inner region and an environment via a germ-impermeable but air-permeable or gas-permeable filter (10) in the closed state of the lid (6),
the ventilating device (12) is configured to be able to convey a medium in the environment of the sterilization container (2) to the media exchange mechanism, and
the control device (14) is configured to control the media exchange mechanism and the ventilating device (12) in such a way that the at least one fluid-mechanical connection (8) is opened when a medium in the inner region is conveyed into the environment and is closed when a medium in the environment is conveyed into the inner region via the germ-impermeable but air-permeable or gas-permeable filter (10).

2. The sterilization container (2) according to claim 1, **characterized in that**
the sterilization container (2) has a sensor device (16) configured to be able to measure pressure, temperature and/or humidity in the inner region of the sterilization container (2) and/or in the environment of the sterilization container (2), and
the control device (14) is configured to control the media exchange mechanism and the ventilating device (12) taking into account one measurement value or several measurement values of the sensor device (16).

3. The sterilization container (2) according to claim 1 or 2, **characterized in that** the media exchange mechanism and the ventilating device (12) are configured on the lid (6).

4. The sterilization container (2) according to one of claims 1 to 3, **characterized in that**
the media exchange mechanism has two fluid-mechanical connections (8) that can each be selectively opened and closed, and
the control device (14) is configured to control the ventilating device (12) in such a way that the ventilating device (12) conveys a medium in the environment of the sterilization container (2) to a first one of the two fluid-mechanical connections (8) and conveys a medium in the inner region of the sterilization container (2) to a second one of the two fluid-mechanical connections (8).

5. The sterilization container (2) according to claim 4, **characterized in that** the control device (14) is configured to be able to control the ventilating device (12) in such a way that the ventilating device (12) alternately conveys a medium in the environment of the sterilization container (2) to the first fluid-mechanical connection (8) as well as conveys a medium in the inner region of the sterilization container (2) to the second fluid-mechanical connection (8) and, on the other hand, a medium in the environment of the sterilization container (2) to the second fluid-mechanical connection (8) as well as a medium in the inner region of the sterilization container (2) to the first fluid-mechanical connection (8).

6. The sterilization container (2) according to one of claims 1 to 5, **characterized by** a wirelessly chargeable energy storage configured to be able to supply the control device (14), the media exchange mechanism, and/or the ventilating device (12) with energy.

7. The sterilization container (2) according to one of claims 1 to 6, **characterized in that** the control device (14) has a receiver configured to receive actuation signals that cause the control device to convey medium out of the inner region and/or out of the environment of the sterilization container (2) via the ventilating device (12).

8. The sterilization container (2) according to one of claims 1 to 7, **characterized in that** the control device (14) comprises a transmitter configured to be able to send status signals containing information about a status of the media exchange mechanism and/or of the ventilating device (12).

9. A system comprising a sterilization container (2) according to claim 7 or 8 and a control apparatus for externally controlling the control device (14) of the sterilization container (2), **characterized by** a transmitter configured to be able to send actuation signals to the control device, and/or a receiver configured to be able to receive status signals from the control device (14).

10. The system according to claim 9, **characterized by** a charging device for wireless charging of an energy storage of a sterilization container (2) according to claim 6.

## Revendications

1. Récipient de stérilisation (2) à usage médical avec
un élément de fond (4),
un couvercle (6) pouvant être fixé à l'élément de fond (4) qui définit une zone intérieure du récipient de stérilisation (2) conjointement avec l'élément de fond (4) dans un état fermé du couvercle (6),
un mécanisme d'échange de milieux,
un appareil de commande (14) et
un appareil de ventilation qui présente un ventilateur (12) et qui est conçu afin de pouvoir acheminer un milieu dans la zone intérieure du récipient de stérilisation (2) vers le mécanisme d'échange de milieux,
**caractérisé en ce que**
le mécanisme d'échange de milieux est conçu afin de pouvoir ouvrir et fermer au choix, dans l'état fermé du couvercle (6), au moins une communication (8) fluidique entre la zone intérieure et un environnement au moyen d'un filtre (10) imperméable aux germes mais perméable à l'air ou aux gaz,
l'appareil de ventilation (12) est conçu afin de pouvoir acheminer un milieu dans l'environnement du récipient de stérilisation (2) vers le mécanisme d'échange de milieux, et
l'appareil de commande (14) est conçu afin de pouvoir commander le mécanisme d'échange de milieux et l'appareil de ventilation (12) de telle manière que la au moins une communication (8) fluidique soit ouverte lors d'un acheminement d'un milieu dans la zone intérieure dans l'environnement et soit fermée lors d'un acheminement d'un milieu dans l'environnement dans la zone intérieure au moyen du filtre imperméable aux germes mais perméable à l'air ou aux gaz.

2. Récipient de stérilisation (2) selon la revendication 1, **caractérisé en ce que**
le récipient de stérilisation (2) présente un appareil de capteur (16) qui est conçu afin de pouvoir mesurer une pression, une température et/ou une humidité dans la zone intérieure du récipient de stérilisation (2) et/ou dans l'environnement du récipient de stérilisation (2) et
l'appareil de commande (14) est conçu afin de pouvoir commander le mécanisme d'échange de milieux et l'appareil de ventilation (12) en tenant compte d'une valeur de mesure ou de plusieurs valeurs de mesure de l'appareil de capteur (16).

3. Récipient de stérilisation (2) selon la revendication 1 ou 2, **caractérisé en ce que** le mécanisme d'échange de milieux et l'appareil de ventilation (12) sont conçus au niveau du couvercle (6).

4. Récipient de stérilisation (2) selon l'une quelconque des revendications 1 à 3, **caractérisé en ce que**
le mécanisme d'échange de milieux présente deux communications (8) fluidiques ouvrables et refermables respectivement au choix et
l'appareil de commande (14) est conçu afin de pouvoir commander l'appareil de ventilation (12) de telle manière que l'appareil de ventilation (12) achemine un milieu dans l'environnement du récipient de stérilisation (2) vers une première des deux communications (8) fluidiques et achemine un milieu dans la zone intérieure du récipient de stérilisation (2) vers une seconde des deux communications (8) fluidiques.

5. Récipient de stérilisation (2) selon la revendication 4, **caractérisé en ce que** l'appareil de commande (14) est conçu afin de pouvoir commander l'appareil de ventilation (12) de telle manière que l'appareil de ventilation (12) achemine alternativement d'un côté un milieu dans l'environnement du récipient de stérilisation (2) vers la première communication (8) fluidique ainsi qu'un milieu dans la zone intérieure du récipient de stérilisation (2) vers la seconde communication (8) fluidique et de l'autre côté achemine un milieu dans l'environnement du récipient de stérilisation (2) vers la seconde communication (8) fluidique ainsi qu'achemine un milieu dans la zone intérieure du récipient de stérilisation (2) vers la première communication (8) fluidique.

6. Récipient de stérilisation (2) selon l'une quelconque des revendications 1 à 5, **caractérisé par** un dispositif de stockage d'énergie rechargeable sans câble qui est conçu afin de pouvoir alimenter en énergie l'appareil de commande (14), le mécanisme d'échange de milieux et/ou l'appareil de ventilation (12).

7. Récipient de stérilisation (2) selon l'une quelconque des revendications 1 à 6, **caractérisé en ce que** l'appareil de commande (14) présente un récepteur qui est conçu afin de pouvoir recevoir des signaux d'actionnement qui incitent l'appareil de commande à acheminer du milieu au moyen de l'appareil de ventilation (12) depuis la zone intérieure et/ou l'environnement du récipient de stérilisation (2).

8. Récipient de stérilisation (2) selon l'une quelconque des revendications 1 à 7, **caractérisé en ce que** l'appareil de commande (14) présente un émetteur qui est conçu afin de pouvoir émettre des signaux de statut qui contiennent une information relative à un statut du mécanisme d'échange de milieux et/ou de l'appareil de ventilation (12).

9. Système avec un récipient de stérilisation (2) selon la revendication 7 ou 8 et un appareil de commande pour la commande externe de l'appareil de commande (14) du récipient de stérilisation (2), **caractérisé par** un émetteur qui est conçu afin de pouvoir envoyer des signaux d'actionnement à l'appareil de commande et/ou un récepteur qui est conçu afin de pouvoir recevoir des signaux de statut de l'appareil de commande (14).

10. Système selon la revendication 9 **caractérisé par** un appareil de charge pour la charge sans câble d'un dispositif de stockage d'énergie d'un récipient de stérilisation (2) selon la revendication 6.
